(19) Europäisches Patentamt
European Patent Office
Office européen des brevets

(11) Publication number: **0 062 470**
**B1**

(12) # EUROPEAN PATENT SPECIFICATION

(45) Date of publication of patent specification: **15.08.84**

(51) Int. Cl.³: **C 07 C 43/21**, C 07 C 13/28, C 09 K 3/34

(21) Application number: **82301631.6**

(22) Date of filing: **29.03.82**

(54) **Liquid crystal benzene derivatives.**

(30) Priority: **02.04.81 JP 49688/81**
**02.04.81 JP 49689/81**
**18.08.81 JP 129070/81**
**03.09.81 JP 138875/81**
**27.10.81 JP 171696/81**

(43) Date of publication of application:
**13.10.82 Bulletin 82/41**

(45) Publication of the grant of the patent:
**15.08.84 Bulletin 84/33**

(84) Designated contracting states:
**CH DE GB LI**

(56) References cited:
**EP-A- 0 022 183**
**US-A- 3 928 481**

(73) Proprietor: **Chisso Corporation**
**6-32, Nakanoshima 3-chome Kita-ku**
**Osaka-shi Osaka-fu (JP)**

(72) Inventor: **Sugimori, Shigeru**
**2493-10, Fujisawa**
**Fujisawashi Kanagawaken (JP)**
Inventor: **Kojima, Tetsuhiko**
**10-3, Otsutomocho Kanazawaku**
**Yokohamashi Kanagawaken (JP)**
Inventor: **Tsuji, Masakazu**
**10-2, Otsutomocho Kanazawaku**
**Yokohamashi Kanagawaken (JP)**

(74) Representative: **Ruffles, Graham Keith et al**
**MARKS & CLERK 57-60 Lincoln's Inn Fields**
**London WC2A 3LS (GB)**

**EP 0 062 470 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to novel liquid crystal substances which exhibit liquid crystal phases within broad temperature ranges and have low viscosities.

Liquid crystal display elements utilize the optical anisotropy and dielectric anisotropy of liquid crystal substances, and are classified into various types such as TN type (twisted nematic type), DS type (dynamic scattering type), guest-host type, DAP type, etc, according to their display modes. The properties of the liquid crystal substances vary according to their respective uses. However, it is common to any liquid crystal that stability to moisture, air, heat, light, etc, are required, and it is also necessary to have a liquid crystal phase within as broad a temperature range as possible, around room temperature, and further, have an optimal value of dielectric anisotropy ($\Delta\varepsilon$) varied depending on the kind of display element. At present, however, no single compound which alone satisfies such conditions is available and it is the present state of the art that a liquid crystal composition obtained by blending several kinds of liquid crystal compound or non-liquid crystal compound is used. Recently, display elements actuating from lower temperatures (about − 20 °C) up to higher temperatures (about 80° to 90 °C) have been particularly needed ; thus, liquid crystal compositions having superior actuation characteristics within a broader temperature range have been desired.

European Patent Application EP-A2-0 022 183 describes cyclohexylbiphenyl compounds

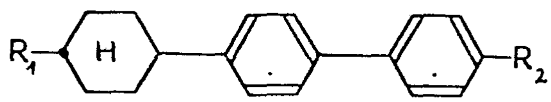

where $R_1$ is $C_{1\text{-}12}$ alkyl and $R_2$ is optionally perfluorinated alkyl, alkoxy or alkanoyloxy, of use in liquid crystal compositions.

An object of the present invention is to provide novel liquid crystal compounds which are useful as components of such liquid crystal compositions and particularly suited for improvement in the low temperature characteristics of display elements.

The present invention is directed to benzene derivatives expressed by the general formula

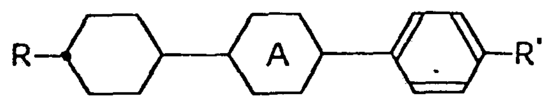 (I)

wherein R represents a hydrogen atom or an alkyl group of 1 to 10 carbon atoms ; R' represents a hydrogen atom, an alkyl group of 1 to 10 carbon atoms or an alkoxy group of 1 to 10 carbon atoms ; and the group

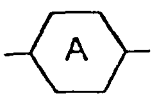

represents a group

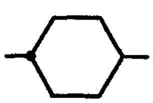

or a group

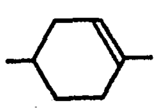

The compounds of the present invention typically exhibit low values of positive dielectric anisotropy have broad liquid crystal temperature ranges ; and in particular, have high liquid crystalline-transparent points (N-I point or Sm-I point), and yet have low viscosities.

Among the compounds of the formula (I), those wherein the group

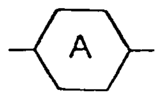

is the group

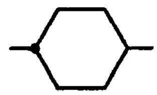

are particularly stable to heat, air, moisture, light, etc ; hence they are extremely useful for preparing liquid crystal compositions actuating over a broad temperature range.

An illustrative process for preparing compounds of the present invention is as follows :

Bromobenzene (in the case where R' is H) or a 4-substituted bromobenzene (in the case where R' is other than H) is first reacted with metallic magnesium in tetrahydrofuran or other solvent to give the corresponding 4-substituded-phenylmagnesium bromide (taking hydrogen at the 4-position to be a substituent), which is then reacted with 4-cyclohexylcyclohexanone (in the case where R is H) or a 4-(trans-4'-alkylcyclohexyl)cyclohexanone (in the case where R is other than H) to give a 4-substituted-[l'-hydroxy-4'-(optionally trans-4"-alkylcyclohexyl)cyclohexyl] benzene, which is then dehydrated, for instance in the presence of potassium hydrogen sulphate as catalyst, to give a 4-substituted-[4'-(cyclohexyl or trans-4"-alkylcyclohexyl)-cyclohexen-1-yl] benzene as one of the objective compounds. This compound can be further reduced, for example in ethanol as solvent in the presence of Raney nickel catalyst under atmospheric pressure and at about 30 °C, to give a 4-substituted-[4'-(cyclohexyl or trans-4"-alkyl-cyclohexyl)-cyclohexyl] benzene. This compound is a mixture of trans-form with cis-form, and can be recrystallized from ethanol to give an objective 4-substituted-[trans-4'-(cyclohexyl or trans-4"-alkylcyclohexyl)cyclohexyl] benzene.

The above reactions can be illustrated by chemical equations as follows :

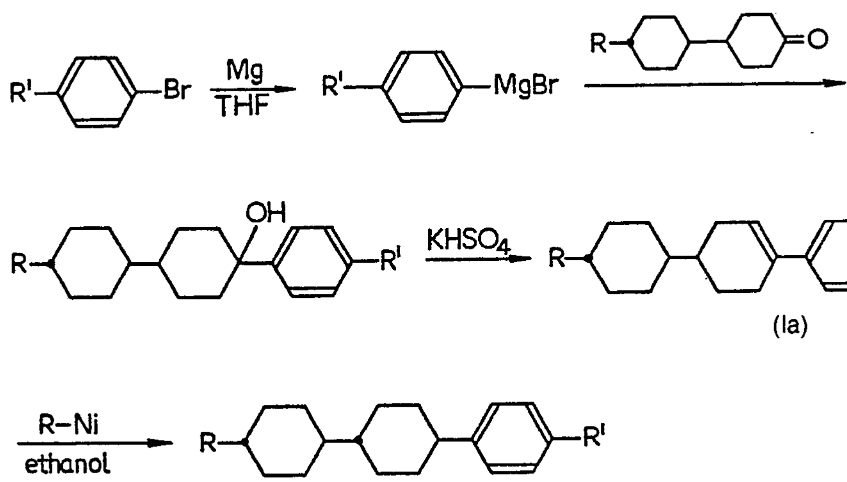


(Ia)

(Ib)

where R and R' are as defined above. The cyclohexanones can be obtained by oxidizing the corresponding cyclohexanols with anhydrous chromic acid.

The process for preparing compounds of the present invention and the use of the compounds will be further described in detail by way of Examples.

Example 1

Preparation of 4-methoxy-[4'-(trans-4"-propylcyclohexyl)-cyclohexen-1'-yl] benzene.

A magnesium shaving (1.2 g, 0.049 mol) was placed in a three-neck flask, and 30 ml of a solution of 4-bromoanisole (9.2 g, 0.049 mol) dissolved in tetrahydrofuran was slowly added dropwise with stirring while passing nitrogen gas through the flask and maintaining the reaction temperature at 30° to 35 °C. As a result of the reaction, the magnesium dissolved in 3 hours to form a uniform solution containing 4-methoxyphenylmagnesium bromide, to which 50 ml of a solution of 4-(trans-4'-propylcyclohexyl)cyclohexanone (10.9 g, 0.049 mol) dissolved in tetrahydrofuran was dropwise added as rapidly as possible while

the reaction temperature was maintained at 5° to 10 °C. After the dropwise addition, the mixture was heated up to 35 °C and agitated for 30 minutes, followed by addition of 50 ml of 3N hydrochloric acid. The reaction liquid was then taken into a separating funnel and subjected to extraction three times with toluene, followed by washing the combined toluene layers with water till it became neutral, and then distilling off the toluene under reduced pressure to give as residue, 4-methoxy-[1′-hydroxy-4′-(trans-4″-propylcyclohexyl)cyclohexyl] benzene.

Potassium hydrogen sulphate (6 g) was added and the mixture was subjected to dehydration in nitrogen gas current at 160 °C for 2 hours. After cooling, toluene (200 ml) was added and potassium hydrogen sulphate was filtered off followed by washing the resulting toluene layer with water till the washing water became neutral. Toluene was then distilled off under reduced pressure to give an oily substance as residue which was recrystallized from ethanol to give the objective 4-methoxy-[4′-(trans-4″-propylcyclohexyl)cyclohexen-1′-yl] benzene. This compound had a crystalline-smectic (C-Sm) point of 95.2 °C, a smectic-nematic (Sm-N) point of 139.2 °C and a nematic-transparent (N-I) point of 207.4 °C. Yield : 7.0 g (46 %). The fact that this product was the objective compound was confirmed by NMR spectra, IR absorption spectra and elementary analysis.

Examples 2 to 61

Example 1 was repeated except that the substituents R and R′ in the above formula (Ia) were varied. The yields (g and %) and physical properties of the resulting compounds are shown in Table 1 together with those for Example 1.

Table 1

Compounds of 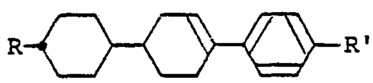 (formula ($I_a$))

| Example | R | R' | Amount of raw material 4-substituted-hexanone used (g) | Yield (g) | Yield (%) | Phase transition points (°C) C-Sm point or C-I point | Sm-N point | N-I point or Sm-I point |
|---|---|---|---|---|---|---|---|---|
| 1 | $C_3H_7$ | $CH_3O$ | 8.7 | 7.0 | 46 | 93.2 | 139.2 | 207.4 |
| 2 | $C_3H_7$ | $C_3H_7O$ | 8.7 | 8.1 | 49 | 87.4 | 187.9 | 200.5 |
| 3 | $C_3H_7$ | $C_7H_{15}O$ | 9.6 | 8.0 | 41 | 70.1 | - | 193.6 |
| 4 | $C_4H_9$ | $C_2H_5O$ | 9.2 | 5.1 | 31 | 81.8 | 182.6 | 202.2 |
| 5 | $C_4H_9$ | $C_6H_{13}O$ | 9.2 | 5.0 | 26 | 72.6 | - | 199.2 |
| 6 | $C_5H_{11}$ | $C_3H_7O$ | 9.8 | 5.2 | 29 | 81.0 | - | 196.8 |
| 7 | $C_6H_{13}$ | $CH_3O$ | 13.0 | 5.5 | 30 | 66.0 | 133.1 | 180.0 |
| 8 | $C_6H_{13}$ | $CH_3$ | 10.3 | 3.0 | 18 | 66.1 | 144.1 | 170.2 |
| 9 | $C_7H_{15}$ | $C_3H_7O$ | 10.9 | 15.4 | 79 | 79.3 | - | 195.9 |
| 10 | $C_7H_{15}$ | $C_7H_{15}O$ | 1.0 | 0.9 | 55 | 79.6 | - | 192.0 |
| 11 | H | H | 7.7 | 5.5 | 43 | 97.7 ~98.4 | - | - |
| 12 | $C_3H_7$ | H | 26.2 | 19.4 | 48 | 68.6 | 77.1 | 105.5 |
| 13 | $C_4H_9$ | H | 9.2 | 4.7 | 32 | 75.8 | 89.0 | 104.8 |

Table 1 (continued)

| Example | R | R' | Amount of raw material 4-substituted-hexanone used (g) | Yield (g) | Yield (%) | Phase transition points (°C) C-Sm point or C-I point | Sm-N point | N-I point or Sm-I point |
|---|---|---|---|---|---|---|---|---|
| 14 | $C_5H_{11}$ | H | 29.5 | 19.5 | 42 | 69.8 | 83.9 | 111.8 |
| 15 | H | $CH_3O$ | 21.3 | 16 | 50 | 115.0 ~116.0 | - | - |
| 16 | H | $C_2H_5O$ | 21.3 | 20 | 60 | 109.8 | - | 119.1 |
| 17 | H | $C_3H_7O$ | 21.3 | 10 | 29 | 100.3 | - | 118.0 |
| 18 | H | $C_4H_9O$ | 21.3 | 19 | 52 | 73.5 | - | 112.1 |
| 19 | H | $C_5H_{11}O$ | 21.3 | 22 | 58 | 73.5 | - | 108.6 |
| 20 | $C_2H_5$ | $CH_3$ | 8.3 | 4.2 | 40 | 74.8 | 130.2 | 151.5 |
| 21 | $C_2H_5$ | $C_2H_5$ | 8.3 | 1.6 | 15 | room temp. or lower | - | 155.9 |
| 22 | $C_2H_5$ | $C_3H_7$ | 8.3 | 1.8 | 15 | room temp. or lower | - | 156.2 |
| 23 | $C_2H_5$ | $C_5H_{11}$ | 8.3 | 1.5 | 8 | room temp. or lower | - | 153.7 |
| 24 | $C_2H_5$ | $C_6H_{13}$ | 8.3 | 2.2 | 15 | room temp. or lower | - | 150.6 |
| 25 | $C_3H_7$ | $CH_3$ | 8.9 | 3.0 | 25 | 80.5 | 133.3 | 180.8 |
| 26 | $C_3H_7$ | $C_3H_7$ | 8.9 | 6.0 | 45 | 57.0 | - | 163.7 |
| 27 | $C_3H_7$ | $C_4H_9$ | 8.9 | 3.0 | 22 | room temp. or lower | - | 176.3 |
| 28 | $C_3H_7$ | $C_5H_{11}$ | 8.9 | 2.7 | 19 | room temp. or lower | - | 175.0 |
| 29 | $C_3H_7$ | $C_6H_{13}$ | 8.9 | 1.1 | 8 | room temp. or lower | - | 173.4 |
| 30 | $C_3H_7$ | $C_2H_5O$ | 13.3 | 9.5 | 29 | 80.0 | 170.0 | 208.5 |
| 31 | $C_3H_7$ | $C_4H_9O$ | 8.7 | 1.6 | 12 | 60.2 | - | 195.6 |
| 32 | $C_3H_7$ | $C_5H_{11}O$ | 6.4 | 2.2 | 21 | 75.8 | 181.0 | 197.2 |
| 33 | $C_3H_7$ | $C_6H_{13}O$ | 10.1 | 4.4 | 25 | 71.3 | - | 192.7 |
| 34 | $C_5H_{11}$ | $CH_3$ | 10.0 | 4.4 | 34 | 70.4 | 141.8 | 174.4 |
| 35 | $C_5H_{11}$ | $C_3H_7$ | 10.0 | 6.7 | 48 | 54.6 | 172.3 | 175.6 |
| 36 | $C_5H_{11}$ | $C_4H_9$ | 10.0 | 1.0 | 7 | room temp. or lower | - | 182.0 |

Table 1 (continued)

| Example | R | R' | Amount of raw material 4-substituted-hexanone used (g) | Yield (g) | Yield (%) | Phase transition points (°C) C-Sm point or C-I point | Sm-N point | N-I point or Sm-I point |
|---|---|---|---|---|---|---|---|---|
| 37 | $C_5H_{11}$ | $C_5H_{11}$ | 10.0 | 6.1 | 40 | room temp. or lower | - | 181.0 |
| 38 | $C_5H_{11}$ | $C_6H_{13}$ | 10.0 | 7.3 | 45 | room temp. or lower | - | 182.0 |
| 39 | $C_5H_{11}$ | $CH_3O$ | 9.8 | 2.5 | 15 | 83.0 | 148.8 | 199.6 |
| 40 | $C_5H_{11}$ | $C_2H_5O$ | 7.3 | 3.0 | 29 | 84.9 | 189.4 | 204.0 |
| 41 | $C_5H_{11}$ | $C_4H_9O$ | 9.8 | 5.6 | 38 | 65.5 | - | 208.3 |
| 42 | $C_5H_{11}$ | $C_5H_{11}O$ | 7.3 | 1.8 | 12 | 75.8 | - | 206.0 |
| 43 | $C_5H_{11}$ | $C_6H_{13}O$ | 7.4 | 7.2 | 62 | 73.6 | - | 203.3 |
| 44 | $C_5H_{11}$ | $C_7H_{15}O$ | 11.4 | 7.4 | 38 | 83.7 | - | 196.8 |
| 45 | $C_7H_{15}$ | $CH_3O$ | 7.5 | 8.0 | 60 | 81.1 | 147.3 | 182.0 |
| 46 | $C_2H_5$ | $C_4H_9$ | 8.3 | 3.5 | 27 | room temp. or lower | - | 150.7 |
| 47 | $C_2H_5$ | $C_7H_{15}$ | 8.3 | 1.0 | 7 | room temp. or lower | - | 139.6 |
| 48 | $C_2H_5$ | $C_8H_{17}$ | 8.3 | 4.4 | 30 | room temp. or lower | - | 148.4 |
| 49 | $C_3H_7$ | $C_7H_{15}$ | 8.9 | 2.8 | 19 | room temp. or lower | - | 165.9 |
| 50 | $C_4H_9$ | $CH_3$ | 9.5 | 6.3 | 51 | 76.7 | 130.1 | 170.4 |
| 51 | $C_4H_9$ | $C_2H_5$ | 9.5 | 4.5 | 35 | 37.3 | - | 163.9 |
| 52 | $C_4H_9$ | $C_3H_7$ | 9.5 | 1.6 | 12 | room temp. or lower | - | 181.6 |
| 53 | $C_4H_9$ | $C_5H_{11}$ | 9.5 | 2.6 | 18 | room temp. or lower | - | 181.8 |
| 54 | $C_5H_{11}$ | $C_7H_{15}$ | 10.0 | 2.0 | 12 | room temp. or lower | - | 174.5 |
| 55 | $C_7H_{15}$ | $CH_3$ | 11.1 | 2.5 | 18 | 67.4 | 147.9 | 174.6 |
| 56 | $C_7H_{15}$ | $C_2H_5$ | 11.1 | 8.2 | 56 | 42.6 | 163.3 | 165.5 |
| 57 | $C_7H_{15}$ | $C_3H_7$ | 11.1 | 9.0 | 59 | 40.8 | - | 171.8 |
| 58 | $C_7H_{15}$ | $C_4H_9$ | 11.1 | 11.6 | 74 | room temp. or lower | - | 179.3 |

(Table 1 (continued)

| Example | R | R' | Amount of raw material 4-substituted-hexanone used (g) | Yield | | Phase transition point (°C) | | |
|---|---|---|---|---|---|---|---|---|
| | | | | (g) | (%) | C-Sm point or C-I point | Sm-N point | N-I point or Sm-I point |
| 59 | $C_7H_{15}$ | $C_5H_{11}$ | 11.1 | 10.0 | 61 | room temp. or lower | - | 182.0 |
| 60 | $C_7H_{15}$ | $C_6H_{13}$ | 11.1 | 9.0 | 53 | room temp. or lower | - | 179.8 |
| 61 | $C_7H_{15}$ | $C_7H_{15}$ | 11.1 | 5.1 | 29 | 49.9 | - | 179.4 |

Example 62

Preparation of 4-methoxy-[trans-4'-(trans-4"-propylcyclohexyl)cyclohexyl] benzene.

4-methoxy-[4'-(trans-4"-propylcyclohexyl)cyclohexen-1'-yl] benzene (7.0 g) prepared in Example 1 was dissolved in ethanol (120 ml) and Raney nickel catalyst (1.0 g) was added, followed by catalytic reduction at 50 °C under atmospheric pressure (quantity of hydrogen absorbed : 500 ml). After filtering off the catalyst, the solution was subjected to recrystallization as it was. The resulting material was a mixture of cis-form with trans-form, and was further repeatedly recrystallized from ethanol to isolate the trans-form. This compound was 4-methoxy-[trans-4'-(trans-4"-propylcyclohexyl)cyclohexyl] benzene, and had a crystalline-smectic (C-Sm) point of 79.2 °C, a smectic-nematic (Sm-N) point of 128.4 °C and a nematic-transparent (N-I) point of 211.5 °C. Yield : 2.8 f (40 %). The fact that this product was the objective compound was confirmed by NMR spectra and elementary analysis.

Examples 63 to 108

Example 62 was repeated except that the substituents were varied. The yields and physical properties of the resulting compounds are shown in Table 2 together with those for Example 62.

Table 2

Compounds of 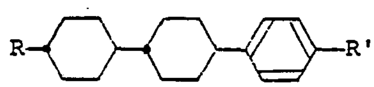 (formula ($I_b$))

| Example | R | R' | Yield (%) | Phase transition points (°C) | | |
|---|---|---|---|---|---|---|
| | | | | C-Sm point or C-I point | Sm-N point | N-I point or Sm-I point |
| 62 | $C_3H_7$ | $CH_3O$ | 40 | 79.2 | 128.4 | 211.5 |
| 63 | $C_3H_7$ | $C_3H_7O$ | 24 | 81.6 | 179.5 | 201.4 |
| 64 | $C_3H_7$ | $C_7H_{15}O$ | 40 | 72.1 | 172.0 | 178.0 |
| 65 | $C_4H_9$ | $C_2H_5O$ | 30 | 84.7 | 172.5 | 211.5 |
| 66 | $C_4H_9$ | $C_6H_{13}O$ | 19 | 70.0 | - | 186.3 |
| 67 | $C_5H_{11}$ | $C_3H_7O$ | 38 | 56.3 | 195.1 | 201.8 |
| 68 | $C_6H_{13}$ | $CH_3O$ | 22 | 70.5 | 154.5 | 196.5 |

Table 2 (continued)

| Example | R | R' | Yield (%) | Phase transition points (°C) C-Sm point or C-I point | Sm-N point | N-I point or Sm-I point |
|---|---|---|---|---|---|---|
| 69 | $C_6H_{13}$ | $CH_3$ | 12 | 135.8 | 142.5 | 167.8 |
| 70 | $C_7H_{15}$ | $C_3H_7O$ | 32 | 62.7 | - | 194.6 |
| 71 | $C_7H_{15}$ | $C_7H_{15}O$ | 20 | 58.1 | - | 188.0 |
| 72 | $C_3H_7$ | H | 23 | 75.9 | 91.8 | 100.4 |
| 73 | $C_4H_9$ | H | 35 | 70.1 | - | 109.2 |
| 74 | $C_5H_{11}$ | H | 15 | 61.6 | 107.9 | 111.8 |
| 75 | H | $CH_3O$ | 31 | 92.8 | - | - |
| 76 | H | $C_2H_5O$ | 25 | 87.0 | - | 98.7 |
| 77 | H | $C_3H_7O$ | 30 | 90.4 | - | 113.0 |
| 78 | H | $C_4H_9O$ | 20 | 85.3 | - | 101.0 |
| 79 | H | $C_5H_{11}O$ | 20 | 62.4 | - | 87.2 |
| 80 | $C_3H_7$ | $CH_3$ | 20 | 64.6 | 109.7 | 179.8 |
| 81 | $C_3H_7$ | $C_2H_5$ | 13 | 31.0 | 161.3 | 162.7 |
| 82 | $C_3H_7$ | $C_3H_7$ | 20 | room temp. or lower | - | 177.5 |
| 83 | $C_3H_7$ | $C_2H_5O$ | 5 | 84.6 | 149.1 | 210.1 |
| 84 | $C_3H_7$ | $C_4H_9O$ | 38 | 65.3 | 179.1 | 190.9 |
| 85 | $C_3H_7$ | $C_5H_{11}O$ | 21 | 60.8 | 175.8 | 185.6 |
| 86 | $C_3H_7$ | $C_6H_{13}O$ | 27 | 77.0 | 175.3 | 185.3 |
| 87 | $C_4H_9$ | $CH_3$ | 33 | 58.0 | 135.2 | 173.9 |
| 88 | $C_5H_{11}$ | $CH_3$ | 39 | 55.8 | 138.0 | 178.6 |
| 89 | $C_5H_{11}$ | $C_2H_5$ | 26 | 37.8 | - | 173.2 |
| 90 | $C_5H_{11}$ | $C_3H_7$ | 16 | 48.6 | - | 181.0 |
| 91 | $C_5H_{11}$ | $C_4H_9$ | 18 | room temp. or lower | - | 190.6 |
| 92 | $C_5H_{11}$ | $CH_3O$ | 30 | 66.3 | 150.1 | 207.7 |
| 93 | $C_5H_{11}$ | $C_2H_5O$ | 27 | 81.8 | 176.3 | 215.2 |

Table 2 (continued)

| Example | R | R' | Yield (%) | Phase transition points (°C) C-Sm point or C-I point | Sm-N point | N-I point or Sm-I point |
|---|---|---|---|---|---|---|
| 94 | $C_5H_{11}$ | $C_4H_9O$ | 7 | 63.2 | 199.2 | 200.6 |
| 95 | $C_5H_{11}$ | $C_5H_{11}O$ | 16 | 61.4 | - | 196.7 |
| 96 | $C_5H_{11}$ | $C_6H_{13}O$ | 10 | 58.1 | - | 192.5 |
| 97 | $C_7H_{15}$ | $CH_3O$ | 22 | 68.8 | 153.0 | 194.7 |
| 98 | $C_2H_5$ | $CH_3$ | 10 | 62.8 | 103.7 | 142.3 |
| 99 | $C_2H_5$ | $C_2H_5$ | 6 | 55.1 | - | 151.9 |
| 100 | $C_2H_5$ | $C_3H_7$ | 9 | room temp. or lower | - | 160.8 |
| 101 | $C_2H_5$ | $C_4H_9$ | 23 | room temp. or lower | - | 158.6 |
| 102 | $C_2H_5$ | $C_5H_{11}$ | 17 | room temp. or lower | - | 151.0 |
| 103 | $C_2H_5$ | $C_6H_{13}$ | 21 | room temp. or lower | - | 148.6 |
| 104 | $C_3H_7$ | $C_4H_9$ | 31 | room temp. or lower | - | 189.6 |
| 105 | $C_3H_7$ | $C_5H_{11}$ | 19 | room temp. or lower | - | 172.2 |
| 106 | $C_3H_7$ | $C_6H_{13}$ | 10 | room temp. or lower | - | 171.0 |
| 107 | $C_5H_{11}$ | $C_5H_{11}$ | 11 | room temp. or lower | - | 189.8 |
| 108 | $C_5H_{11}$ | $C_6H_{13}$ | 7 | room temp. or lower | - | 185.9 |

Next, use examples will be described. Parts referred to herein are by weight.

Example 109 (Use Example 1)

A liquid crystal composition (A) consisting of

trans-4-propyl-(4'-cyanophenyl)cyclohexane (28 %),
trans-4-pentyl-(4'-cyanophenyl)cyclohexane (43 %),
trans-4-heptyl-(4'-cyanophenyl)cyclohexane (29 %),

has a nematic temperature range of − 3 to + 52 °C, a dielectric anisotropy value Δε of + 10.5, a threshold voltage of 1.53 V and a saturation voltage of 2.12 V. Also it has a viscosity of 23 cp (0.023 Pa.s) at 20 °C.

To this liquid crystal composition A (80 parts) were added 4-methoxy-[4'-(trans-4''-propylcyclohexyl)cyclohexen-1'-yl] benzene (10 parts) prepared in Example 4 to give a liquid crystal mixture. This mixture had a greatly extended a nematic temperature range of − 10 to + 81 °C. Its Δε was reduced down to 8.8 and its threshold voltage and saturation voltage were elevated up to 1.63 V and 2.28 V, respectively. Nevertheless, the viscosity (23 cp, 0.023 Pa.s) was unchanged.

Example 110 (Use Example 2)

To the above liquid crystal composition (A) (90 parts) was added 4′-(trans-4″-pentylcyclohexyl)cyclohexen-1′-ylbenzene 10 parts prepared in Example 14 to give a liquid crystal mixture. This mixture had as extended a nematic temperature range as − 5 to + 58 °C. The Δε, threshold voltage and saturation voltage were elevated up to + 9.0 V, 1.60 V, and 2.30 V, respectively but the viscosity (23 cp, 0.023 Pa.s) was unchanged.

Example 111 (Use Example 3)

To the liquid crystal composition (A) (80 parts) were added 4-methoxy-[trans-4′-(trans-4″-propylcyclohexyl)cyclohexyl] benzene (10 parts) prepared in Example 62 and 4-methoxy-[trans-4′-hexylcyclohexyl)cyclohexyl] benzene (10 parts) prepared in Example 68 to give a liquid crystal mixture. This mixture had as extended a nematic temperature range as − 10 to + 78 °C. The Δε, was reduced down to + 8.8 and the threshold voltage and saturation voltage were elevated up to 1.63 V, and 2.28 V, respectively but its viscosity (23 cp, 0.023 Pa.s) was unchanged. Thus it is seen that the compounds of the present invention are effective for lower temperature actuation.

Example 112 (Use Example 4)

To the liquid crystal composition (A) (60 parts) were added [trans-4′-(trans-4″-propylcyclohexyl)cyclohexyl] benzene (20 parts) prepared in Example 72 and [trans-4′-(trans-4″-pentylcyclohexyl)cyclohexyl] benzene (20 parts) prepared in Example 74 to give a liquid crystal mixture. This mixture had as extended a nematic temperature range as − 5 to + 73 °C. The Δε was reduced down to + 7.2 and the threshold voltage and saturation voltage were somewhat elevated up to 1.75 V, and 2.30 V, respectively but the viscosity (23 cp, 0.023 Pa.s) was unchanged. Thus, by adding the compounds of the present invention, it was possible to extend the nematic temperature range with slight increases in the threshold voltage and saturation voltage in spite of reduction in the dielectric anisotropy value.

Example 113 (Use Example 5)

A liquid crystal composition (B) consisting of

| | |
|---|---|
| 4-pentyl-4′-cyanobiphenyl | 45 % |
| 4-heptyl-4′-cyanobiphenyl | 29 % |
| 4-octyloxy-4′-cyanobiphenyl | 15 % |
| 4-pentyl-4′-cyanoterphenyl | 11 % |

had a N-I point of 63.3 °C and a viscosity of 48 cp (0.048 Pa.s) at 20 °C.

To this composition (B) (95 parts) was added 1-(trans-4′-cyclohexylcyclohexyl)-4-propoxybenzene (5 parts) prepared in Example 77 to give a liquid crystal mixture having a N-I point of 61.8 °C and a viscosity of 47.5 cp (0.047 5 Pa.s) at 20 °C.

Example 114 (Use Example 6)

A composition (C) consisting of of

| | |
|---|---|
| 4-pentyl-4′-cyanobiphenyl | 51 % |
| 4-heptyl-4′-cyanobiphenyl | 32 % |
| 4-octyloxy-4′-cyanobiphenyl | 17 % |

had a N-I point of 44.3 °C and a viscosity of 40 cp (0.04 Pa.s) at 20 °C. To this composition (C) (90 parts) was added 1-(trans-4′-cyclohexylcyclohexyl)-4-methoxybenzene (10 parts) prepared in Example 75 to give a liquid crystal mixture having a N-I point of 44.1 °C and a viscosity of 39 cp (0.039 Pa.s) at 20 °C.

**Claims**

1. A benzene derivative expressed by the general formula

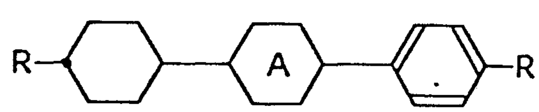

(I)

wherein R represents a hydrogen atom or an alkyl group of 1 to 10 carbon atoms ; R′ represents a

hydrogen atom, an alkyl group of 1 to 10 carbon atoms or an alkoxy group of 1 to 10 carbon atoms ; and the group

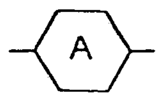

represents a group

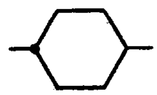

or a group

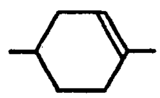

2. A [trans-4′-(trans-4″-cyclohexyl)cyclohexyl] benzene expressed by the general formula

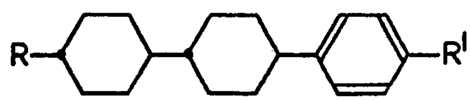

(Ib)

wherein R represents a hydrogen atom or an alkyl group of 1 to 10 carbon atoms and R′ represents a hydrogen atom, an alkyl group of 1 to 10 carbon atoms or an alkoxy group or 1 to 10 carbon atoms.

3. A [4′-(trans-4″-cyclohexyl)cyclohexen-1′-yl] benzene expressed by the general formula

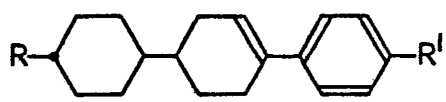

(Ia)

wherein R represents a hydrogen atom or an alkyl group of 1 to 10 carbon atoms and R′ represents a hydrogen atom, an alkyl group of 1 to 10 carbon atoms or an alkoxy group or 1 to 10 carbon atoms.

4. A liquid crystal composition which contains at least one benzene derivative according to any of claims 1 to 3.

5. A liquid crystal display cell employing a composition according to claim 4.

**Ansprüche**

1. Benzol-Derivate der allgemeinen Formel

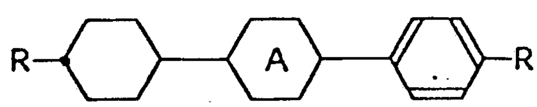

(I)

wobei R = H oder eine Alkyl-Gruppe mit 1 bis 10 Kohlenstoffatomen, R′ = H, eine Alkyl-Gruppe mit 1 bis 10 Kohlenstoffatomen oder eine Alkoxy-Gruppe mit 1 bis 10 Kohlenstoffatomen und die Gruppe

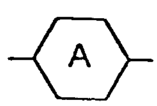 = 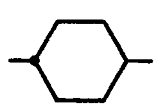 oder 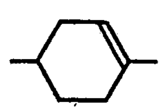

2. Ein [trans-4′-(trans-4″-Cyclohexyl)cyclohexyl]-benzol der allgemeinen Formel

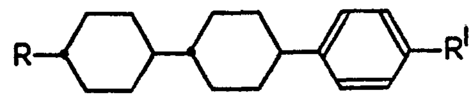

(Ib)

wobei R = H oder eine Alkyl-Gruppe mit 1 bis 10 Kohlenstoffatomen und R' = H, eine Alkyl-Gruppe mit 1 bis 10 Kohlenstoffatomen oder eine Alkoxy-Gruppe mit 1 bis 10 Kohlenstoffatomen.

3. Ein [4'-(trans-4"-Cyclohexyl)cyclohexen-1'-yl]-benzol der allgemeinen Formel

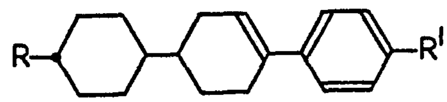

(Ia)

wobei R = H oder eine Alkyl-Gruppe mit 1 bis 10 Kohlenstoffatomen und R' = H, eine Alkyl-Gruppe mit 1 bis 10 Kohlenstoffatomen oder eine Alkoxy-Gruppe mit 1 bis 10 Kohlenstoffatomen.

4. Eine Flüssigkristall-Mischung, enthaltend wenigstens ein Benzol-Derivat nach einem der Ansprüche 1 bis 3.

5. Eine Flüssigkristall-Anzeigezelle mit einer Mischung nach Anspruch 4.

**Revendications**

1. Dérivé du benzène exprimé par la formule générale

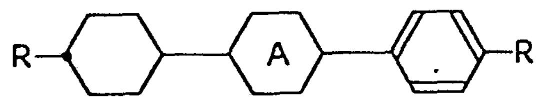

(I)

où R = H ou un groupe alkyle de 1 à 10 atomes de carbone, R' = H, un groupe alkyle de 1 à 10 atomes de carbone ou un groupe alkoxy de 1 à 10 atomes de carbone et le groupe

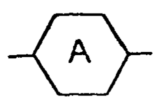 = 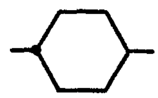 ou 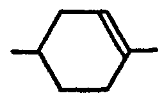

2. [trans-4'-(trans-4"-Cyclohexyl)cyclohexyl]-benzène exprimé par la formule générale

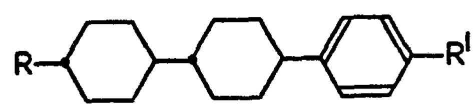

(Ib)

où R = H ou un groupe alkyle de 1 à 10 atomes de carbone et R' = H, un groupe alkyle de 1 à 10 atomes de carbone ou un groupe alkoxy de 1 à 10 atomes de carbone.

3. [4'-(trans-4"-Cyclohexyl)cyclohexène-1'-yl] benzène exprimé par la formule générale

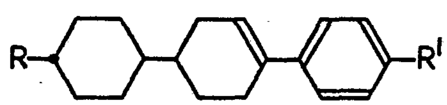

(Ia)

où R = H ou un groupe alkyle de 1 à 10 atomes de carbone et R' = H, un groupe alkyle de 1 à 10 atomes de carbone ou un groupe alkoxy de 1 à 10 atomes de carbone.

4. Mélange de cristaux liquides comprenant au moins un dérivé du benzène, selon l'une quelconque des revendications 1 à 3.

5. Cellule d'affichage à cristaux liquides utilisant un mélange selon la revendication 4.